# EUROPÄISCHE PATENTSCHRIFT

(11) **EP 0 713 380 B1**
(45) Veröffentlichungstag und Bekanntmachung des Hinweises auf die Patenterteilung: **05.09.2001**
(21) Anmeldenummer: 94923659.0
(22) Anmeldetag: 12.08.1994
(51) Int. Cl.: A61K 7/00, A61K 7/48

(54) **FUNKTIONELLES SAUERSTOFFHALTIGES PRÄPARAT ENTHALTEND PHOSPHOLIPIDE UND FLUORCARBON**
FUNCTIONAL OXYGENATED COMPOSITION CONTAINING PHOSPHOLIPIDS AND FLUOROCARBON
PREPARATION FONCTIONNELLE OXYGENEE CONTENANT DES PHOSPHOLIPIDES ET DU FLUOROCARBONE

(30) Priorität: 13.08.1993 DE 4327679
(43) Veröffentlichungstag der Anmeldung: 29.05.1996
(73) Patentinhaber: LANCASTER GROUP GmbH, 55116 Mainz (DE)
(72) Erfinder: ZASTROW, Leonhard, MC-98000 Monaco (MC); GOLZ, Karin, D-13137 Berlin (DE); STANZL, Klaus, White Plains, NY 10605 (US)
(74) Vertreter: Walter, Wolf-Jürgen
(86) Internationale Anmeldenummer: DE9400943
(87) Internationale Veröffentlichungsnummer: WO9505144

(56) Entgegenhaltungen:
- EP-A- 0 472 225
- WO-A-94/00098
- DE-A- 4 127 442

## Beschreibung

Die Erfindung betrifft ein Präparat, das bestimmte Nähr-, Wirk- und Schutzstoffe enthält sowie einen Sauerstoffträger. Es betrifft weiterhin ein Verfahren zur Herstellung dieses Präparates und dessen Verwendung.

Es ist bekannt, bestimmte Wirkstoffe aus Pflanzen oder auch aus Hefen zu gewinnen und sie in der Kosmetik oder Dermatologie einzusetzen. Dazu gehören u.a. Proteine wie Superoxiddismutase (DE-OS 2417508; EP-A-19474). Es ist auch bereits vorgeschlagen worden, pflanzliche Inhaltsstoffe in Liposomenstrukturen zu verkapseln und auf die Haut aufzubringen.

Ferner sind kürzlich kosmetische und dermatologische Produkte vorgeschlagen worden, die mit Hilfe mit Phospholipiden mit hohem Phosphatidylcholingehalt und Fluorcarbonen neuartige asymmetrische lamellare Strukturen ausbilden und dabei hohe Sauerstoffgehalte in die oberen Hautschichten infolge guter Penetrierungseigenschaften einbringen können. Fluorcarbone sind synthetische Produkte, deren Herstellung erhebliche Kosten verursacht und die zur besseren Wirksamkeit in Mengen von 10 bis 40 % in kosmetischen Zubereitungen enthalten sein können.

Der Erfindung liegt die Aufgabe zugrunde, Hautpräparate mit sowohl hohem Sauerstoffgehalt als auch einem Gehalt an weiteren Nähr-, Wirk- und/oder Schutzstoffen bei gleichzeitiger Kostensenkung bereitzustellen.

Erfindungsgemäß besteht das neue funktionelle sauerstoffhaltige Präparat aus
a) Phospholipide und mit Sauerstoff beladenes Fluorcarbon oder Fluorcarbongemisch, wobei der Anteil an Fluorcarbon im Bereich von 0,2 bis 100 % Gewicht/Volumen liegt, mit einem Phosphatidylcholingehalt der Lipidfraktion von 30 bis 99 Gewichts-%, in Form von asymmetrischen lamellaren Aggregaten,
b) einem durch schonenden Aufschluß mittels Ultraschall und/oder Hochdruckhomogenisierung bis 25 MPa von Suspensionen oder Dispersionen von Zellen pflanzlicher Stoffe, Bakterien oder Hefen erhaltenen Aufschlußprodukt, und
c) einem für die Anwendung auf der Haut geeigneten kosmetischen oder dermatologischen Trägerstoff.

Es wurde überraschenderweise gefunden, daß bei gleichzeitigem Vorhandensein von asymmetrischen lamellaren Aggregaten aus Fluorcarbonen und Phospholipiden mit hohem Phosphatidylcholingehalt einerseits und andererseits mit von diesen Aggregaten getragenen Aufschlußprodukten von Zellen pflanzlicher Stoffe oder Hefen eine Sauerstoffmenge in den Aggregaten vorliegt, die höher ist, als die durch den Fluorcarbongehalt und die damit einzustellende kritische Löslichkeitstemperatur zu erwarten ist. Dieser synergistische Effekt ermöglicht eine Verringerung des Fluorcarbongehaltes bei gleichzeitiger Einführung nützlicher Nähr-, Wirk- und Schutzstoffe aus dem Aufschluß der pflanzlichen und Hefezellen.

Bei dem Präparat werden als pflanzliche Stoffe vorteilhaft Grünalgen, Samen, Körner, Rinden, Pflanzenextrakte eingesetzt, bei deren schonendem Aufschluß der Zellen solche Stoffe entstehen wie Proteine, Enzyme, Nukleinsäuren, Vitamine, Hormone, Fluoranoide, Flavanoide usw. Ein besonders vorteilhafter pflanzlicher Stoff für die erfindungsgemäße Präparation ist die Rinde des mexikanischen Hautbaumes, dessen Aufschlußprodukte noch nicht vollständig aufgeklärt sind, die jedoch in Kombination mit Fluorcarbonen oder Fluorcarbongemischen überraschend hohe Sauerstoffgehalte und entzündungshemmende Wirkung in einer Präparation aufweisen.

Besonders wertvoll sind auch solche Kombinationen, bei denen hohe Säureanteile von solchen Säuren wie Fruchtsäuren, z.B. Äpfelsäure, Citronensäure, Weinsäure, Fumarsäure, Bersteinsäure, Gluconsäure sowie Milchsäure mit Fluorcarbonen zu auf die Haut aufzubringenden Präparaten verarbeitet werden.

Ebenfalls wertvoll sind Kombinationen mit Vitaminen, wie einem oder mehreren der Gruppe Vitamin A, Vitamin B, Vitamin C, Vitamin D, Vitamin E, Vitamin P sowie mit Flavonoiden, d.h. den pharmakologisch wirksamen Glycosiden der Flavone. Dazu gehören insbesondere auch die Bioflavonoide, die auch als Vitamin-P-Faktoren bezeichnet werden, z.B. Rutin, aber auch andere Flavonoide wie beispielsweise Flavonol, Chrysin, Galangin, Apigenin, Fisetin, Luteolin, Kämpferol, Quercetin, Morin und deren Derivate in Form biologisch wirksamer Substanzen.

Erfindungsgemäß besonders wirksame Präparate sind auch solche, die zusätzlich zu dem erhöhten Sauerstoffgehalt Wirkstoffe in Form von Vitaminen mit den Vitaminkombinationen P-B-A oder A-E-C oder B-E-A enthalten.

Als Hefen sind für den Aufschluß besonders geeignet Bäkkerhefen, Bierhefen, Weinhefen sowie Superoxiddismutase-angereicherte Hefen.

Als Bakterien sind für den Aufschluß besonders geeignet solche der Klasse Phycomycetes wie Phytophthora cactorum; Ascomycetes wie Asperillus niger K1, Chaetomium globosum, Penicillinum chrysogenum; Basidomycetes wie Coniophora cerebella, Corticium confluens ; Deuteromycetes wie Gloeosporium fructigenum, Fusarium oxysporum, Alternaria solani usw. Die Aufzählung ist beispielhaft.

Zur Herstellung der asymmetrischen lamellaren Aggregate können eine Vielzahl von Fluorcarbonen eingesetzt werden, z.B. aliphatische geradkettige und verzweigte Fluoralkane, mono- oder bicyclische und gegebenenfalls fluoralkylsubstituierte Fluorcycloalkane, perfluorierte aliphatische oder bicyclische Amine, Bis-(perfluoralkyl)-Ethene, Perfluorpolyether oder deren Gemische. Besonders bevorzugt sind solche Fluorcarbone wie Perfluordecalin, F-Butyltetrahydrofuran, Perfluortributylamin, Perfluoroctylbromid, Bis-Fluor(Butyl)ethen oder Bis-Fluor(hexyl)ethen oder C₆-C₉-Perfluoralkane. Dabei liegt der Anteil an Fluorcarbonen im Bereich von 20 bis 100 % w/v, vorzugsweise im Bereich von 40 bis 100 %. Ein besonders bevorzugter Bereich ist der von 70 bis 100 % w/v.

Unter dem hier verwendeten Begriff "Fluorcarbone" werden perfluorierte oder hochfluorierte Kohlenstoffverbindungen oder Gemische verstanden, die in der Lage sind, Gase wie O₂ und CO₂ zu transportieren. Hochfluorierte Kohlenwasserstoffverbindungen sind im Sinne dieser Erfindung solche, bei denen die meisten Wasserstoffatome durch Fluoratome ersetzt sind, so daß bei weiterem Ersatz nicht notwendigerweise die Fähigkeit zum Gas-transport erhöht wird. Dies wird meist dann erreicht, wenn etwa bis zu 90 % der Wasserstoffatome durch Fluoratome ersetzt sind. Bevorzugt im Sinne der vorliegenden Erfindung sind Fluorcarbone, bei denen wenigstens 95 % der Wasserstoffatome ersetzt sind, bevorzugter 98 % und am bevorzugtesten 100 %.

Als Phospholipide werden natürliche Phospholipide wie Sojalecithin und Eilecithin, synthetische Phospholipide sowie auch hydrierte Lecithine, z.B. Phospholipone H oder teilhydrierte Phospholipide eingesetzt. Bei diesen Phospholipiden liegt der Gehalt an Phosphatidylcholin bei 30 bis 99 % und insbesondere bei 70 bis 90 %.

Neben Phosphatidylcholin können auch Lysolecithine im Konzentrationsbereich von 0,1 bis 10 Gew.-% und/oder geladene Phospholipide wie Phosphatidylethanolamin, n-Acetylphosphatidylethanolamin oder Phosphatidsäure im Konzentrationsbereich 0,1 bis 30 Gew.-% vorhanden sein.

Im Unterschied zu den bekannten wäßrigen Liposomen (Vesikel) tragen diese Phospholipid-stabilisierten Aggregate in ihrem Kern hydrophobe Fluorcarbone, die zum Transport von Sauerstoff befähigt sind. Ihre grenzflächenchemische Stabilisierung erfolgt primär durch eine Monolayer mit inverser Anordnung und gegebenenfalls ein sich daran anschließender Aufbau von Bilayer-Schichten. Wegen der Besonderheit ihrer strukturellen Anordnung werden diese neuartigen Aggregate als asymmetrische lamellare Sauerstoff-Carrier bezeichnet. Ihre außergewöhnliche kolloidchemische Stabilität ist vermutlich auf die lamellare Struktur und auf die Oberflächenladung der Aggregate zurückzuführen. Letztere ist auf die Auswahl geeigneter Phospholipide beziehungsweise deren Mischungen natürlicher wie auch synthetischer Provenienz zurückzuführen. In erster Linie sind für eine vorteilhafte Wirkung in diesem Sinne Phospholipide, insbesondere Phosphatidylcholin im genannten Konzentrationsbereich von 30 bis 99 % in Verbindung mit Lysolecithinen der Konzentration von 0,1 bis 10 % und/oder geladenen Phospholipiden im Konzentrationsbereich 0,1 bis 30 Gew.-% verantwortlich. Die angesprochene Wirkung der Phospholipide wird durch entsprechende negative Zeta-Potentiale und durch die Messung von Ladungsdichten (bei Titration mit einem kationischen Polyelektrolyten) verifiziert. Wesentlich für den Einsatz der Fluorcarbon-Aggregate ist die Hautpenetrierung in Abhängigkeit von der kritischen Löslichkeitstemperatur der aus gewählten Fluorcarbone oder Fluorcarbongemische (s.a. DE-A-42 21 255).

Es kann auch eine wäßrige Fluorcarbonemulsion mit einem Gehalt an 1 bis 8 Masse-% eines nichtionogenen Tensids als Emulgator verwendet werden, für die ein besonders vorteilhafter Gehalt an Fluorcarbonen im Bereich von 40 bis 100 % (w/v), insbesondere 70 bis 100 % liegt. Als nichtionogenes Tensid können eingesetzt werden perfluorierte Imino-bis(polyoxyalkylene), Polyoxyethylen-Polyoxypropylen-Copolymere, ethoxylierte Sorbitanfettsäureester, nichtionogene ethoxylierte Fluortenside und/oder ethoxylierte Polypropylenglycole.

Ein besonders vorteilhaftes Aufschlußprodukt rührt aus einem Ultraschallaufschluß mit einer Ultraschall-Durchflußzelle her, bei der die Synotrode zu ½ bis % ihrer Länge in die Durchflußzelle hineinragt, der Winkel der Synotrode im Beschallungsgefäß im Bereich von 80,5 bis 88,5 ° liegt, das Verhältnis der Eintauchlänge der Synotrode (in mm) zum Beschallungsvolumen (in ml) auf einen Wert im Bereich von 1:1,1 bis 1:20 eingestellt ist und das Verhältnis von Eintauchlänge der Synotrode (in mm) zu dem Feststoffanteil des zu beschallenden Mediums (in Masse-%) im Bereich von 1:0,02 bis 1:2,2 liegt.

Die überraschende Wirkung der Kombination von asymmetrischen lamellaren Aggregaten und den Aufschlußprodukten pflanzlicher und Hefezellen ist besonders ausdrucksvoll beim Aufschluß von Superoxiddismutase-reichen Hefen, die ein hohen Gehalt an Superoxiddismutase (SOD) liefern. Da SOD als Radikalfänger in der Haut wirkt und die Reaktion

2 O₂⁻ + 2 H⁺

H₂O₂ + O₂

katalysiert, ist es per se ein besonders vorteilhafter Bestandteil in kosmetischen oder dermatologischen Präparaten. Es können jedoch auch andere Radikalfänger verwendet werden, die sich zur Bindung freier Sauerstoffradikale eignen, beispielsweise Vitamin E, P.

Eine weitere vorteilhafte Präparation besteht darin, daß das pflanzliche Aufschlußprodukt aus Zellen des mexikanischen Hautbaumes hergestellt wird, wodurch ein besonders entzündungshemmendes sauerstoffreiches Produkt erhalten wird.

Die Erfindung betrifft auch ein Verfahren zur Herstellung des funktionellen sauerstoffhaltigen Präparates. Das Verfahren ist dadurch gekennzeichnet, daß
(a) Suspensionen oder Dispersionen von Zellen pflanzlicher Stoffe, Bakterien oder Hefen durch schonende Ultraschallbehandlung und/oder Hochdruckhomogenisierung bis 25 MPa aufgeschlossen werden,
(b) Phospholipide mit einem sauerstoffbeladenen Fluorcarbon oder Fluorcarbongemisch in wäßrigen Medium emulgiert werden, wobei der Fluorcarbonanteil im Bereich von 0,2 bis 100 % Gewicht/Volumen liegt und der Gehalt an Phosphatidylcholin 30 bis 99 Masse-% beträgt,
(c) das Aufschlußprodukt von (a) mit der Emulsion (b) vermischt wird, und
(d) die bei (c) erhaltenen asymmetrischen lamellaren Aggregate, die die Aufschlußprodukte (a) tragen, in einen für die Anwendung auf der Haut geeigneten Träger eingearbeitet werden. Dabei kann das Fluorcarbongemisch auch eine Fluorcarbonemulsion mit nichtionogenen Tensiden sein, wie oben näher ausgeführt.

Es ist besonders vorteilhaft, den Zellaufschluß durch Ultraschall nach dem in der DE-Patentanmeldung 42 41 154.8 beschriebenen Verfahren durchzuführen. Dieses Verfahren führt durch die besondere Anordnung der Synotrode in einer Ultraschall-Durchflußzelle zu einem rationellen und schonendem Aufschluß der Zellen und gewährleistet optimale Ausbeuten an nützlichem Aufschlußprodukten wie Proteinen, Vitaminen, Enzymen usw. Dabei können Feststoffkonzentrationen in den zu beschallenden Medium im Bereich von 0,5 bis 65 Masse-% vorliegen. Ein besonders vorteilhafter Synotrodenwinkel beträgt 85,3 °. Die Amplitude liegt vorzugsweise im Bereich von 20 bis 70.

Eine weitere Möglichkeit zum Aufschluß der Zellen besteht in der Hochdruckhomogenisierung, die bis zur Anwendung eines Druckes von etwa 25 MPa (etwa 250 atü) durchgeführt werden kann. Weiterhin ist es auch möglich, beide Verfahren in besonderen Fällen miteinander zu kombinieren.

Die Erfindung betrifft auch die Verwendung des funktionellen sauerstoffhaltigen Präparates mit den Inhaltsstoffen, wie sie oben beschrieben wurden, zur gleichzeitigen Versorgung der Haut mit hohen Sauerstoffgehalten und mit einem oder mehreren Stoff beziehungsweise Stoffen der Gruppe Nährstoffe, Wirkstoffe, Schutzstoffe und pharmazeutisch wirksame Stoffe. Diese Stoffe können für die Haut und/oder das darunter liegende Gewebe wirksam werden.

Entsprechende Formulierungen können mit einem für die kosmetische bzw. dermatologische Anwendung üblichen Träger zu Salben, Cremes, Lotionen, Wässern, alkoholischen Auszügen, Pasten, Pudern, Gelen, Tinkturen usw. zu Hautpflegemitteln, Sonnenschutzformulierungen mit UV-Absorbern, Bräunungsmitteln, nachfettenden After-Shaves, Reinigungslotionen und -ölen, verkapselten Radikalfängern, Formulierungen gegen Schwangerschaftsstreifen, Haar- und Haarbodenpflegemitteln, Ölbädern, Fitneß-Friktionen usw. verarbeitet werden.

Dazu können gegebenenfalls weitere pharmazeutisch wirksame Stoffe in die Formulierung eingebracht werden. Dazu gehören beispielsweise
pharmakologische Wirkstoffe in Form von dermatologischen systemischen Wirkstoffen, einschließlich Cytostatika, Cancerostatika, Immunmodulatoren und Vakzinen, insbesondere solche der folgenden Gruppe: dermatologische Wirkstoffe, wie zum Beispiel Virustatika oder viruzide Arzneistoffe, Antimykotika, Heparine (z.B. Heparin-Calcium, Heparin-Natrium, niedermolekulare Heparine), Antibiotika, Corticoide, Antiinfektiosia, Aknewirkstoffe, Lokalanästhetika, Antiphlogistika, Antihistaminika oder Antipsoriatika;
systemische Wirkstoffe, wie zum Beispiel nichtsteroidale Analgetika/Antirheumatika (z.B. Diclofenac-Natrium, Diclofenac-Diethylaminsalz, Etofenamat, Flufenaminsäure, 2-Hydroxyethylsalicylat, Ibuprofen, Indomethacin, Piroxicam), Opiatrezeptor-Agonisten und -Antagonisten (z.B. Buprenorphin, Fentanyl, Pentazocin, Pethidin, Tilidin, Tramadol, Naloxon), Histaminantagonisten (z.B. Bamipinlactat, Chlorphenoxamin-HCl, Clemastinhydrogenfumarat, Dimetindenmaleat, Pheniraminhydrogenmaleat), Insuline, regulatorische Peptide und ihre Hemmstoffe (z.B. Hypophysenvorderlappenhormone und ihre Hemmstoffe, Hypophyenhinterlappenhormone, Hypothalamushormone), Sedativa/ Hypnotika (z.B. Diazepam);
Wirkstoffe der Gruppe Cytostakika, Cancerostatika, Immunmodulatoren, Vakzine.

Ein bevorzugter dermatologischer Wirkstoff ist beispielsweise Rosmarinsäure oder ein anderer in Pflanzen vorkommender viruzider oder virustatischer Wirkstoff. Ein bevorzugter systemischer Wirkstoff ist beispielsweise ein niedermolekulares oder hochmolekulares Heparin, ein Oligopeptid oder ein Polypeptid.

Weitere bevorzugte Wirkstoffe sind Vitamine (E, A, B, C), Muramylpeptide, Doxorubicin, Gentamycin, Gramycidin, Dexamethason, Hydrocortison, Progesteron, Prednisolon bzw. davon abgeleitete Derivate und/oder Säure- bzw. Basenadditionssalze.

Den oben aufgeführten dermatologischen Wirkstoffen können erforderlichenfalls ein oder mehrere Anti-Oxidationsmittel hinzugesetzt werden.

Die Erfindung soll nachstehend durch Beispiele näher erläutert werden.

### Beispiel 1

### Herstellung der Fluorcarbonemulsion

50 ml einer 10%igen wäßrigen Phospholipidlösung (Sojalecithin, 40 % Phosphatidylcholin (PC)) werden gemeinsam mit 80 g eines hochreinen, keine H-Atome enthaltenden Fluorcarbongemisches (90 % Perfluordecalin, 10 % F-Dibutylmethylamin, kritische Löslichkeitstemperatur 26 °C) mit einem Ultraschalldesintegrator unter Eiskühlung homogenisiert bis die Teilchengröße der Partikel einen mittleren Durchmesser von 244 nm aufweisen. Aus ³¹P-NMR-Messungen ist anhand der typischen Signalbreite wie auch aus elektronenmikroskopischen Aufnahmen die lamellare Struktur der Aggregate aus Fluorcarbon und Phospholipid zu erkennen.
Die Aggregatdispersion läßt sich unproblematisch und ohne Beeinflussung ihrer Stabilität mit geeigneten Alkoholen (Ethanol, Propylenglycol, Glycerol) zum Zweck der Sterilisierung mischen. Ein Zusatz von 30 ml Ethanol erzeugt Keimfreiheit, wobei die resultierende Dispersion folgende Zusammensetzung aufweist:

| | |
|---|---|
| 62 | % w/v Fluorcarbone |
| 9,7 | % Phospholipide |
| 19 | % Ethanol |

Das Zeta-Potential von minus 61 mV belegt eine durch die Phospholipide erzeugte negative Oberflächenladung mit einer elektrostatischen Stabilisierung der Dispersion.

### Herstellung des Aufschlußproduktes von Hefen

| | |
|---|---|
| 23,5 | Masse-% Bäckerhefe |
| 10,0 | Masse-% Glycerin |
| 5,5 | Masse-% Propylenglycol |
| q.s. | destilliertes Wasser. |

Das destillierte Wasser wird bei 5 bis 7 °C in einen Behälter vorgelegt. Unter Rühren wird die Hefe darin dispergiert. Danach werden das Glycerin und das Propylenglycol zu der Suspension hinzugesetzt.

Die homogene Hefesuspension wird mittels einer Pumpe durch ein Ultraschall-Durchflußgerät geleitet und dort der Ultraschallbehandlung aufgesetzt. Dabei erfolgt ein schonender Zellaufschluß unter Gewinnung der aktiven Zellinhaltsstoffe (z.B. Proteine wie Zn + Cu-Superoxiddismutase; Vitamine wie Vitamin-B-Komplex, Vitamin A, Vitamin E. Die Parameter in der Zelle waren folgende:

| | |
|---|---|
| Amplitude | 55 |
| Synotrodenwinkel | 85,3 ° |
| Durchflußgeschwindigkeit | 1 l/h |
| Gesamtvolumen des Durchflußbehälters | 550 ml |
| Synotrodenlänge im Gefäß | 30 mm |
| Feststoffanteil | 23,5 Masse-% |
| Aufschlußquote | 95 - 99 % |

Das Verhältnis von Synotrodenlänge : Volumen : Feststoffanteil betrug 1 : 18 : 0,8. Die Gesamtlänge der Synotrode betrug 50 mm. Das Verhältnis von Synotrodenlänge im Gefäß zur Gesamtlänge betrug daher 0,6.

### Die Herstellung des kosmetischen Präparates

Die aufgeschlossene Hefesuspension wurde zentrifugiert. 30 % des Zentrifugates gelangen in die Fluorcarbonaggregate. Die Kombination wurde als Wirkstoffkomplex der O/W-Emulsion zugegeben.

| O/W-Emulsion | |
|---|---|
| Phase A: | |
| Glycerylstearat | 3,5 % |
| Stearinsäure | 2,0 % |
| Cetylakohol | 2,0 % |
| Dimenthinocon | 1,5 % |
| | |

| Phase B: | |
|---|---|
| destillierte Wasser | q.s. |
| Carbomer | 0,7 % |
| Propylenglycol | 3,0 % |
| Konservierungsmittel | 0,5 % |

| Phase C: | |
|---|---|
| TEA-Wirkstoffkomplex mit aufgeschlossener Hefe | 10,0% |

Die Phasen A und B wurden separat auf 75 °C erwärmt und homogen vermischt. Bei < 40 °C wurde der Wirkstoffkomplex unter Rühren zugegeben.

### Beispiel 2

Anstelle des Hefe-Aufschlußproduktes aus Beispiel 1 wurde ein Aufschlußprodukt von Rinde des mexikanischen Hautbaumes hergestellt mit folgender Zusammensetzung

| | |
|---|---|
| 35 | Masse-% Hautbaum, pulverisiert |
| 5,0 | Masse-% Glycerin |
| 5,0 | Masse-% Propylenglycol |

q.s. destilliertes Wasser. Die Verarbeitung erfolgte bei 15 % in ähnlicher Weise wie in Beispiel 1. Dabei wurde der Synotrodenwinkel auf 87,0 ° eingestellt, die Amplitude auf 65, die Synotrodenlänge im Gefäß auf 33,2 mm und das Volumen des Durchflußbehälters betrug bei einer Durchflußgeschwindigkeit von 0,5 l/h 650 ml. Man erhielt eine Aufschlußquote von 96 % bei einem eingesetzten Feststoffanteil von 35 Masse-%. Das Verhältnis von Synotrodenlänge : Volumen : Feststoffanteil betrug 1 : 19 : 1. Bei gleicher Synotrodenlänge wie im Beispiel 1, betrug das Verhältnis von Synotrodenlänge im Gefäß zur Gesamtlänge 0,664.

Die asymmetrischen lamellaren Aggregate auf Basis von Perfluordecalin wurden mit diesem Aufschlußprodukt zu einem kosmetischen Präparat in folgender Weise vermischt.

30 % des Zentrifugates gelangen in die Fluorcarbonaggregate und wurden unter gleichen Bedingungen wie in Beispiel 1 in die O/W-Emulsion eingearbeitet.
Weiterhin wurde ein Hefeaufschluß in die Präparation eingearbeitet so daß sich ein Gemisch im Verhältnis
Hefe in Fluorcarbonaggregat : mexikanischer Hautbaum in Fluorcarbonaggregat wie 2 : 1 ergab.
Aus der folgenden Tabelle 1 ist der O₂-Gehalt in der O/W-Emulsion bei Zusatz der Wirkstoffkomponenten ersichtlich.

**Tabelle 1**

| Erzeugnis | Sauerstoffgehalt bei 20 °C/ppm | | |
|---|---|---|---|
| | sofort nach Herstellung | nach 24 h | nach 4 Wochen |
| O/W-Grundlage (nach Beispiel) ohne Wirkstoffe | 15,5 | 15,9 | 15,0 |
| O/W (nach Beispiel) mit Fluorcarbonaggregat 25 % | 65,6 | 65,0 | 64,9 |
| O/W (nach Beispiel) mit 10 % aufgeschlossenem Hefezentrifugat | 17,9 | 18,2 | 18,5 |
| O/W (nach Beispiel) mit 10 % der Wirkstoffkombination; Fluorcarbonaggregat + 10 % aufgeschlossenem Hefezentrifugat | 62,5 | 65,0 | 66,2 |
| O/W (nach Beispiel) mit 10 % der Wirkstoffkombination; Fluorcarbonaggregat + 10 % aufgeschlossenen mexikanischen Hautbaumzentrifugat | 18,0 | 17,8 | 17,6 |
| O/W (nach Beispiel) mit 10 % der Wirkstoffkombination: Fluorcarbonaggregat und aufgeschlossenem Hefe-/Hautbaumzentrifugat im Verhältnis 2:1 = 10 % | 65,8 | 79,5 | 79,9 |

Aus Tabelle 1 ergibt sich deutlich, daß bereits bei 10 % Fluorcarbonaggregat und 10 % Aufschlußprodukt des mexikanischen Hautbaumes eine deutliche Steigerung des Sauerstoffgehaltes auf einen Wert eintritt, der nahe dem eines 25 %igen Gehaltes von Fluorcarbonaggregat liegt. Dies ist ein klarer Beweis für den synergistischen Effekt, der auch durch das 2:1-Gemisch von Hefe und Hautbaumzentrifugat gestützt wird.

## Patentansprüche

1. Funktionelles sauerstoffhaltiges Präparat, gekennzeichnet durch einen Gehalt an
a) Phospholipide und mit Sauerstoff beladenes Fluorcarbon oder
* , bezogen auf die Fluorcarbonemulsion, Fluorcarbongemisch, wobei der Anteil an Fluorcarbon
* im Bereich von 0,2 bis 100 % Gewicht/Volumen liegt, mit einem Phosphatidylcholingehalt der Lipidfraktion von 30 bis 99 Gewichts-%, in Form von asymmetrischen lamellaren Aggregaten mit einer Hautpenetrierung in Abhängigkeit von der kritischen Löslichkeitstemperatur der Fluorcarbone,
b) einem Produkt des schonenden Aufschlusses durch Ultraschall und/oder Hochdruckhomogenisierung von Suspensionen oder Dispersionen von Zellen pflanzlicher Stoffe, Bakterien oder Hefen, und
c) einem für die Anwendung auf der Haut geeigneten kosmetischen oder dermatologischen Trägerstoff.

2. Präparat nach Anspruch 1, **dadurch gekennzeichnet**, daß die Hefe Bäckerhefe, Bierhefe, Weinhefe, Superoxiddismutase-angereicherte Hefe ist.

3. Präparat nach Anspruch 1, **dadurch gekennzeichnet**, daß der pflanzliche Stoff die Rinde des mexikanischen Hautbaumes ist.

4. Verfahren nach Anspruch 1, **dadurch gekennzeichnet**, daß der pflanzliche Stoff ausgewählt ist unter Grünalgen, Samen, Körner, Rinden, Pflanzenextrakte.

5. Präparat nach Anspruch 1, **dadurch gekennzeichnet**, daß das Aufschlußprodukt einen oder mehrere Stoffe der Gruppe Proteine wie Superoxiddismutase, Enzyme, Nukleinsäure, Vitamine, Fluoranoide, Hormone, Naturstoffe wie Aloe Vera, Rosmarin, Kamille enthält.

6. Präparat nach Anspruch 1, **dadurch gekennzeichnet**, daß das Aufschlußprodukt Kombinationen von Fruchtsäuren, wie Äpfelsäure, Citronensäure, Weinsäure, Fumarsäure, Bersteinsäure, Gluconsäure sowie Milchsäure enthält.

7. Präparat nach Anspruch 1, **dadurch gekennzeichnet**, daß das Aufschlußprodukt Vitamine mit den Vitaminkombinationen P-B-A oder A-E-C oder B-E-A enthält.

8. Präparat nach Anspruch 1, **dadurch gekennzeichnet**, daß die Fluorcarbone aus der Gruppe ausgewählt sind, die aus aliphatischen geradkettigen und verzweigten Fluoralkanen, mono- oder bicyclischen gegebenenfalls fluoralkylsubstituierten Fluorcycloalkanen, perfluorierten aliphatischen oder bicyclischen Aminen, Bis-(perfluoralkyl)ethenen oder deren Gemischen besteht, vorzugsweise aus Fluorcarbonen der Gruppe Perfluordecalin, F-Butyltetrahydrofuran, Perfluortributylamin, Perfluoroctylbromid, Bisfluor(butyl)ethen oder C₆-C₉-Perfluoralkanen.

9. Präparat nach Anspruch 1(a), **dadurch gekennzeichnet**, daß der Anteil an Fluorcarbonen im Bereich von 20 bis 100 % Gewicht/Volumen liegt, vorzugsweise im Bereich von 40 bis 100 %, bezogen auf die Fluorcarbonemulsion.

10. Präparat nach Anspruch 1, **dadurch gekennzeichnet**, daß es zusätzlich pharmakologische Wirkstoffe in Form von dermatologischen systemischen Wirkstoffen, einschließlich Cytostatika, Cancerostatika, Immunmodulatoren und Vakzinen, insbesondere solche der folgenden Gruppe: dermatologische Wirkstoffe, wie zum Beispiel Virustatika oder viruzide Arzneistoffe, Antimykotika, Heparine (wie Heparin-Calcium, Heparin-Natrium, niedermolekulare Heparine), Antibiotika, Corticoide, Antiinfektiosia, Aknewirkstoffe, Lokalanästhetika, Antiphlogistika, Antihistaminika oder Antipsoriatika;
systemische Wirkstoffe, wie nichtsteroidale Analgetika/Antirheumatika (wie Diclofenac-Natrium, Diclofenac-Diethylaminsalz, Etofenamat, Flufenaminsäure, 2-Hydroxyethylsalicylat, Ibuprofen, Indomethacin, Piroxicam), Opiatrezeptor-Agonisten und - Antagonisten (z.B. Buprenorphin, Fentanyl, Pentazocin, Pethidin, Tilidin, Tramadol, Naloxon), Histaminantagonisten (wie Bamipinlactat, Chlorphenoxamin-HCl, Clemastinhydrogenfumarat, Dimetindenmaleat, Pheniraminhydrogenmaleat), Insuline, regulatorische Peptide und ihre Hemmstoffe (wie Hypophysenvorderlappenhormone und ihre Hemmstoffe, Hypophyenhinterlappenhormone, Hypothalamushormone), Sedativa/ Hypnotika (wie Diazepam); Wirkstoffe der Gruppe Cytostakika, Cancerostatika, Immunmodulatoren, Vakzine enthält.

11. Verfahren zur Herstellung eines funktionellen sauerstoffhaltigen Präparates, **dadurch gekennzeichnet**, daß
(a) Suspensionen oder Dispersionen von Zellen pflanzlicher Stoffe, Bakterien oder Hefen durch schonende Ultraschallbehandlung und/oder Hochdruckhomogenisierung aufgeschlossen werden,
(b) Phospholipide mit einem sauerstoffbeladenen Fluorcarbon oder Fluorcarbongemisch in wäßrigen Medium emulgiert werden, wobei der Fluorcarbonanteil, bezogen auf die Fluorcarbonemulsion, im Bereich von 0,2 bis 100 % Gewicht/Volumen liegt und der Gehalt an Phosphatidylcholin in der Lipidfraktion 30 bis 99 Masse-% beträgt,
(c) das Aufschlußprodukt von (a) mit der Emulsion (b) vermischt wird, und
(d) die bei (c) erhaltenen asymmetrischen lamellaren Aggregate, die die Aufschlußprodukte (a) tragen, in einen für die Anwendung auf der Haut geeigneten Träger eingearbeitet werden.

12. Verfahren nach Anspruch 11, **dadurch gekennzeichnet**, daß das Aufschlußprodukt aus einem Ultraschallaufschluß mit einer Ultraschall-Durchflußzelle herrührt, bei der die Synotrode zu ½ bis % ihrer Länge in die Durchflußzelle hineinragt, der Winkel der Synotrode im Beschallungsgefäß im Bereich von 80,5 bis 88,5 ° liegt, das Verhältnis der Eintauchlänge der Synotrode (in mm) zum Beschallungsvolumen (in ml) auf einen Wert im Bereich von 1:1,1 bis 1:20 eingestellt ist und das Verhältnis von Eintauchlänge der Synotrode (in mm) zu dem Feststoffanteil des zu beschallenden Mediums (in Masse-%) im Bereich von 1:0,02 bis 1:2,2 liegt.

13. Verfahren nach Anspruch 12, **dadurch gekennzeichnet**, daß die Feststoffkonzentration im zu beschallenden Medium im Bereich von 0,5 bis 65 Masse-% liegt.

14. Verfahren nach Anspruch 11, **dadurch gekennzeichnet**, daß die Teilchengröße der asymmetrischen lamellaren Aggregate im Bereich von 50 bis 1000 nm liegt, vorzugsweise im Bereich von 120 bis 820 nm, insbesondere im Bereich von 140 bis 400 mm.

15. Kosmetische Verwendung eines funktionellen sauerstoffhaltigen Präparates, bestehend aus
a) Phospholipide und mit Sauerstoff beladenes Fluorcarbon oder Fluorcarbongemisch, wobei der Anteil an Fluorcarbon im Bereich von 0,2 bis 100 % Gewicht/Volumen liegt, mit einem Phosphatidylcholingehalt der Lipidfraktion von 30 bis 99 Gewichts-%, in Form von asymmetrischen lamellaren Aggregaten,
b) einem Aufschlußprodukt durch Ultraschall und/oder Hochdruckhomogenisierung von Suspensionen oder Dispersionen von Zellen pflanzlicher Stoffe, Bakterien oder Hefen, und
c) einem für die Anwendung auf der Haut geeigneten kosmetischen Trägerstoff
zur gleichzeitigen Versorgung der Haut mit Sauerstoff und einem oder mehreren Stoff(en) der Gruppe Nährstoffe und Schutzstoffe für die Haut und/oder das darunter liegende Gewebe.

16. Verwendung nach Anspruch 15, **dadurch gekennzeichnet**, daß es mit den entsprechenden Trägerstoffen in Form von Hautpflegemitteln, Sonnenschutzformulierungen mit UV-Absorbern, Bräunungsmitteln, nachfettenden After-Shaves, Reinigungslotionen und -ölen, verkapselten Radikalfängern, Formulierungen gegen Schwangerschaftsstreifen, Haar- und Haarbodenpflegemitteln, Ölbädern, Fitneß-Friktionen eingesetzt wird.

17. Verwendung eines funktionellen sauerstoffhaltigen Präparates zur Herstellung einer pharmazeutische wirksamen Zusammensetzung, **dadurch gekennzeichnet**, daß das Präparat aus folgenden Komponenten besteht:
a) Phospholipide und mit Sauerstoff beladenes Fluorcarbon oder Fluorcarbongemisch, wobei der Anteil an Fluorcarbon im Bereich von 0,2 bis 100 % Gewicht/Volumen liegt, mit einem Phosphatidylcholingehalt der Lipidfraktion von 30 bis 99 Gewichts-%, in Form von asymmetrischen lamellaren Aggregaten,
b) einem Aufschlußprodukt durch Ultraschall und/oder Hochdruckhomogenisierung von Suspensionen oder Dispersionen von Zellen pflanzlicher Stoffe, Bakterien oder Hefen, und
c) einem für die Anwendung auf der Haut geeigneten dermatologischen Trägerstoff
zur gleichzeitigen Versorgung der Haut mit Sauerstoff und einem oder mehreren Stoff(en) der Gruppe Nährstoffe, Schutzstoffe und pharmazeutisch wirksame Stoffe gemäß Anspruch 10 für die Haut und/oder das darunter liegende Gewebe.

## Claims

1. Functional oxygenaceous preparation, comprising a content of
a) phospholipids and an oxygen laden fluorocarbon or fluorocarbon mixture, the proportion of fluorocarbon, in relation to the fluorocarbon emulsion, being in the range of 0,2 to 100% weight/volume, and comprising a phosphatidylcholine content of the lipid fraction of 30 to 99% by weight in the form of asymmetrical lamellar aggregates, having a skin penetration which is a function of the critical solubility temperature of the fluorocarbons.
b) a product obtained by the mild digestive treatment by means of ultrasonics and/or high pressure homogenisation of suspensions or dispersions of cells of vegetable matter, bacteria or yeasts, and
c) a cosmetic or dermatological carrier substance suitable for application to the skin.

2. A preparation according to claim 1, wherein the yeast is bakers' yeast, brewers' yeast, wine yeast or yeast enriched with peroxide dismutase.

3. A preparation according to claim 1, wherein the vegetable matter is the bark of the Mexican skin tree.

4. A preparation according to claim 1, wherein the vegetable matter is selected from green algae, seeds, grains, barks, plant extracts.

5. A preparation according to claim 1, wherein the digestion product contains one or more substances of the group of proteins such as peroxide dismutase, enzymes, nucleic acids, vitamins, fluoranoids, hormones, or natural substances such as Aloe vera, rosemarin, camomile.

6. A preparation according to claim 1, wherein the digestion product contains combinations of fruit acids such as malic acid, citric acid, tartaric acid, fumaric acid, succinic acid, gluconic acid as well as lactic acid.

7. A preparation according to claim 1, wherein the digestion product contains vitamins comprising the vitamin combination P-B-A or A-E-C or B-E-A.

8. A preparation according to claim 1, wherein the fluorocarbons are selected from the group consisting of aliphatic straight chain and branched chain fluoroalkanes, mono- or bicyclic and optionally fluoroalkyl substituted fluorocycloalkanes, perfluorinated aliphatic or bicyclic amines, bis-(perfluoroalkyl)-ethenes, perfluoropolyethers or their mixtures, preferably from fluorocarbons of the group of perfluorodecalin, F-butyltetrahydrofuran, perfluorotributylamine, perfluoroctylbromide, bis-fluoro(butyl)ethene or C₆-C₉ perfluoroalkanes.

9. A preparation according to claim 1(a), wherein the proportion of fluorocarbons is in the range of 20 to 100% w/v, based on fluorocarbon emulsion, preferably in the range of 40 to 100%.

10. A preparation according to claim 1, wherein it comprises additionally pharmacological active substances in the form of dermatological systemic active substances, including cytostatics, cancerostatics, immunomodulators and vaccines, in particular those of the following group: dermatological active substances such as, for example, virostatics or virocidal medications, anti-mycotics, heparines (such as heparin calcium, heparin sodium, low molecular weight heparines), antibiotics, corticoides, anti-infectives, anti-acne agents, local anaesthetics, antiphlogistics, antihistamines or antipsoriatics;
systemic-active agents such as, for example, non-steroidal analgesics/anti-rheumatics (e.g. diclofenacsodium, diciofenacdiethylamine salt, etofenamate, flufenamin acid, 2-hydroxyethylsalicylate, ibuprofene, indomethacine, piroxicam), opiatereceptor-agonists and -antagonists (e.g. buprenorphine, fentanyle, pentazocin, pethidin, tilidin, tramadol, naloxon), histamine antagonists (e.g. bamipinlactate, chlorophenoxamine-HCl, clemastinhydrogenfumarate, dimetindenmaleate, pheniraminhydrogenmaleate), insulines, regulatory peptides and their inhibitors (e.g. adenohypophyse hormones and their inhibitors, neurohypophyse hormones, hypothalamus hormones), sedatives/hypnotics (e.g. diazepam); active substances of the group cytostatics, cancerostatics, immunmodulators, vaccines.

11. Process for the manufacture of a functional oxygenaceous preparation, wherein
(a) suspensions or dispersions of cells of vegetable matter, bacteria or yeasts are digestively treated by mild ultrasonic treatment and/or high pressure homogenisation,
(b) phospholipids are emulsified with an oxygen-laden fluorocarbon or fluorocarbon mixture in an aqueous medium, the fluorocarbon content being in the range of 0,2 to 100% weight/volume and the content of phosphatidylcholine in the lipid fraction amounting to 30 to 99 wt %.
(c) the digestive treatment product of (a) being mixed with the emulsion (b), and
(d) the asymmetrical lamellar aggregates obtained by (c) which incorporate the digestive treatment products (a) are incorporated in a carrier suitable for application to the skin.

12. Process according to claim 11, wherein the digestive treatment product is obtained by an ultrasonic digestive treatment using an ultrasonic continuous flow cell in which the sonotrode projects as to 1/2 to 2/3 its length into the continuous flow cell, the angle of the sonotrode in the sound exposure vessel being in the range of 80.5 to 88.5°, the ratio of immersion length of the sonotrode (in mm) to the sound irradiated volume (in ml) being set to a value in the range of 1:1.1 to 1:20 and the ratio of immersion length of the sonotrode (in mm) to the solids content of the medium to be irradiated ultrasonically (in wt %) being in the range of 0:0.02 to 1:2.2.

13. Process according to claim 12, wherein the solids concentration present in the medium to be subjected to ultrasonic treatment is in the range of 0.5 to 65 wt%.

14. Process according to claim 11, wherein the particle size of the asymmetrical lamellar Aggregates is in the range of 50 to 1000 nm, preferably in the range of 120 to 820 nm, especially in the range of 140 to 400 nm.

15. Cosmetic use of a functional oxygenaceous preparation, comprising
a) phospholipids and an oxygen laden fluorocarbon or fluorocarbon mixture, the proportion of fluorocarbon being in the range of 0.2 to 100 % weight/volume, and comprising a phosphatidylcholine content of the lipid fraction of 30 to 99% by weight in the form of asymmetrical lamellar aggregates,
b) a product obtained by digestive treatment by means of ultrasonics and/or high pressure homogenisation of suspensions or dispersions of cells of vegetable matter, bacteria or yeasts, and
c) a cosmetic carrier substance suitable for application to the skin
for the simultaneous supply of the skin with oxygen and with one or more substances of the group nutrients and protective agents for the skin and/or the tissue underneath the skin.

16. Use according to claim 15, which is practised with appropriate carrier substances in the form of skin-care agents, sun protection formulations with UV absorbers, tanning agents, fat replenishing after-shaves, cleansing lotions and oils, encapsulated radical scavengers, formulations against pregnancy stretch marks, hair and scalp care agents, bath oils, fitness friction agents.

17. Use of a functional oxygenaceous preparation for preparing a pharmaceutical effective composition, which preparation comprises the following components:
a) phospholipids and an oxygen laden fluorocarbon or fluorocarbon mixture, the proportion of fluorocarbon being in the range of 0.2 to 100 % weight/volume, and comprising a phosphatidylcholine content of the lipid fraction of 30 to 99% by weight in the form of asymmetrical lamellar aggregates,
b) a product obtained by digestive treatment by means of ultrasonics and/or high pressure homogenisation of suspensions or dispersions of cells of vegetable matter, bacteria or yeasts, and
c) a dermatological carrier substance suitable for application to the skin
for the simultaneous supply of the skin with oxygen and with one or more substances of the group nutrients, protective agents and pharmaceutically active substances for the skin and/or the tissue underneath the skin.

## Revendications

1. Préparation contenant de l'oxygène fonctionnel, caractérisée par une teneur :
a) en phospholipides et en fluorocarbone ou mélange de fluorocarbones chargés en oxygène, la proportion de fluorocarbone, ramenée à l'émulsion de fluorocarbone, se situant dans la plage de 0,2 à 100% en poids/volume et la fraction lipidique ayant une teneur en phosphatidylcholine dans la plage de 30 à 99% en poids, sous la forme d'agrégats lamellaires asymétriques avec une pénétration dans la peau en fonction de la température de solubilité critique des fluorocarbones,
b) en un produit obtenu en désagrégeant avec ménagement aux ultrasons et/ou par homogénéisation à haute pression des suspensions ou des dispersions de cellules de matières végétales, de bactéries ou de levures, et
c) en un véhicule cosmétique ou dermatologique approprié pour l'application sur la peau.

2. Préparation selon la revendication 1, **caractérisée en ce que** la levure est de la levure de boulanger, de la levure de bière, de la levure de vin, de la levure enrichie en superoxyde dismutase.

3. Préparation selon la revendication 1, **caractérisée en ce que** la matière végétale est l'écorce de l'arbre à peau mexicain.

4. Préparation selon la revendication 1, **caractérisée en ce que** la matière végétale est choisie parmi des algues vertes, des semences, des graines, des écorces, des extraits de plantes.

5. Préparation selon la revendication 1, **caractérisée en ce que** le produit de désagrégation contient une ou plusieurs substances du groupe des protéines telles que la superoxyde dismutase, des enzymes, de l'acide nucléique, des vitamines, des fluoranoïdes, des hormones, des substances naturelles telles que l'Aloe vera, le romarin, la camomille.

6. Préparation selon la revendication 1, **caractérisée en ce que** le produit de désagrégation contient des combinaisons d'acides de fruits tels que l'acide malique, l'acide citrique, l'acide tartrique, l'acide fumarique, l'acide succinique, l'acide gluconique ainsi que l'acide lactique.

7. Préparation selon la revendication 1, **caractérisée en ce que** le produit de désagrégation contient des vitamines avec les combinaisons de vitamines P-B-A ou A-E-C ou B-E-A.

8. Préparation selon la revendication 1, **caractérisée en ce que** les fluorocarbones sont choisis dans le groupe comprenant des fluoroalcanes aliphatiques à chaîne droite ou ramifiée, des fluorocycloalcanes mono- ou bicycliques et le cas échéant substitués par un fluoroalkyle, des amines perfluorées aliphatiques ou bicycliques, des bis-(perfluoroalkyl)-éthènes, des polyéthers perfluorés ou des mélanges de ceux-ci, de préférence des fluorocarbones choisis dans le groupe comprenant la perfluorodécaline, le F-butyltétrahydrofurane, la perfluorotributylamine, le bromure de perfluorooctyle, le bis-fluoro-(butyl)éthène ou le bis-fluoro(hexyl)éthène ou des perfluoroalcanes en C₆-C₉.

9. Préparation selon la revendication 1(a), **caractérisée en ce que** la proportion de fluorocarbones se situe dans la plage de 20 à 100% en poids/volume, de préférence dans la plage de 40 à 100%, ramenée à l'émulsion de fluorocarbone.

10. Préparation selon la revendication 1, **caractérisée en ce qu**'elle contient en plus des principes actifs pharmacologiques sous la forme de principes actifs dermatologiques systémiques, y compris des cytostatiques, des carcinostatiques, des immunomodulateurs et des vaccins, en particulier ceux appartenant au groupe suivant : des principes actifs dermatologiques tels que des virustatiques ou des médicaments virulicides, des antimycosiques, des héparines (par exemple l'héparine calcium, l'héparine sodium, des héparines de bas poids moléculaire), des antibiotiques, des corticoïdes, des anti-infectieux, des antiacnéiques, des anesthésiques locaux, des antiphlogistiques, des anti-histaminiques ou des anti-psoriasiques ;
des principes actifs systémiques tels que des analgésiques/antirhusmatisma.ux non stéroïdes (par exemple le diclofénac sodium, le diclofénac sel de diéthylamine, l'étofénamate, l'acide flufénamique, le salicylate de 2-hydroxyéthyle, l'ibuprofène, l'indométhacine, le piroxicam), des agonistes et des antagonistes des récepteurs morphiniques (par exemple la buprénorphine, le fentanyl, la pentazocine, la péthidine, la tilidine, le tramadol, la naloxone), des antagonistes de l'histamine (par exemple le lactate de bamipine, la chlorphénoxamine-HCl, l'hydrogénofumarate de clémastine, le maléate de dimétindène, l'hydrogénomaléate de phéniramine), des insulines, des peptides régulateurs et leurs inhibiteurs (par exemple des hormones du lobe antérieur de l'hypophyse et leurs inhibiteurs, des hormones du lobe postérieur de l'hypophyse, des hormones de l'hypothalamus), des sédatifs/hypnotiques (par exemple le diazépam) ;
des principes actifs du groupe des cytostatiques, des carcinostatiques, des immunomodulateurs, des vaccins.

11. Procédé pour fabriquer une préparation contenant de l'oxygène fonctionnel, **caractérisé en ce que** :
(a) on désagrège des suspensions ou des dispersions de cellules de matières végétales, de bactéries ou de levures en les traitant avec ménagement aux ultrasons et/ou par homogénéisation à haute pression,
(b) on émulsionne des phospholipides avec un fluorocarbone ou mélange de fluorocarbones chargé en oxygène dans un milieu aqueux, la teneur en fluorocarbone se situant dans la plage de 0,2 à 100% en poids/volume et la fraction lipidique ayant une teneur en phosphatidylcholine dans la plage de 30 à 99% en masse,
(c) on mélange le produit de désagrégation de l'étape (a) avec l'émulsion (b), et
(d) on incorpore les agrégats lamellaires asymétriques obtenus en (c), qui renferment les produits de désagrégation (a), dans un véhicule approprié pour l'application sur la peau.

12. Procédé selon la revendication 11, **caractérisé en ce que** le produit de désagrégation provient d'une désagrégation aux ultrasons utilisant une cuvette de sonication à flux continu dans laquelle la sonotrode dépasse de 1/2 à 2/3 de sa longueur dans la cuvette à flux continu, l'angle de la sonotrode dans le récipient de sonication se situe dans la plage de 80,5 à 88,5°, le rapport de la longueur d'immersion de la sonotrode (en mm) sur le volume de sonication (en ml) est ajusté à une valeur dans la plage de 1:1,1 à 1:20 et le rapport de la longueur d'immersion de la sonotrode (en mm) sur la proportion de solides dans le milieu à traiter aux ultrasons (en % en masse) se situe dans la plage de 1:0,02 à 1:2,2.

13. Procédé selon la revendication 12, **caractérisé en ce que** la concentration en solides dans le milieu à traiter aux ultrasons se situe dans la plage de 0,5 à 65% en masse.

14. Procédé selon la revendication 11, **caractérisé en ce que** la granulométrie des agrégats lamellaires asymétriques se situe dans la plage de 50 à 1000 nm, de préférence dans la plage de 120 à 820 nm, en particulier dans la plage de 140 à 400 mm.

15. Utilisation cosmétique d'une préparation contenant de l'oxygène fonctionnel, composée :
a) de phospholipides et de fluorocarbone ou d'un mélange de fluorocarbones chargé en oxygène, la proportion de fluorocarbone, ramenée à l'émulsion de fluorocarbone, se situant dans la plage de 0,2 à 100% en poids/volume et la fraction lipidique ayant une teneur en phosphatidylcholine dans la plage de 30 à 99% en poids, sous la forme d'agrégats lamellaires asymétriques,
b) d'un produit obtenu en désagrégeant avec ménagements par sonication et/ou par homogénéisation à haute pression des suspensions ou des dispersions de cellules de matières végétales, de bactéries ou de levures, et
c) d'un véhicule cosmétique approprié pour l'application sur la peau,
pour alimenter simultanément la peau en oxygène et en une ou plusieurs substance(s) du groupe des substances nutritives et des substances protectrices pour la peau et/ou le tissu sous-jacent.

16. Utilisation selon la revendication 15, **caractérisée en ce que** la préparation est utilisée avec les véhicules correspondants sous la forme de produits de soins de la peau, de formulations de protection solaire avec des absorbeurs d'UV, de produits de bronzage, d'après-rasage regraissants, de lotions et d'huiles nettoyantes, de piégeurs de radicaux encapsulés, de formulations anti-vergetures, de produits de soins des cheveux et du cuir chevelu, de bains d'huiles, de frictions de culturisme.

17. Utilisation d'une préparation contenant de l'oxygène fonctionnel pour fabriquer une composition pharmaceutiquement efficace, **caractérisée en ce que** la préparation comprend les composants suivants :
a) des phospholipides et un fluorocarbone ou un mélange de fluorocarbones chargé en oxygène, la proportion de fluorocarbone, ramenée à l'émulsion de fluorocarbone, se situant dans la plage de 0,2 à 100% en poids/volume et la fraction lipidique ayant une teneur en phosphatidylcholine dans la plage de 30 à 99% en poids, sous la forme d'agrégats lamellaires asymétriques,
b) un produit obtenu en désagrégeant avec ménagement aux ultrasons et/ou par homogénéisation à haute pression des suspensions ou des dispersions de cellules de matières végétales, de bactéries ou de levures, et
c) un véhicule dermatologique approprié pour l'application sur la peau,
pour alimenter simultanément la peau en oxygène et en une ou plusieurs substance(s) du groupe des substances nutritives, des substances protectrices et des substances pharmaceutiquement efficaces selon la revendication 10 pour la peau et/ou le tissu sous-jacent.
